(19) Europäisches Patentamt
European Patent Office
Office européen des brevets

(11) **EP 4 407 571 A1**

(12) **EUROPEAN PATENT APPLICATION**
published in accordance with Art. 153(4) EPC

(43) Date of publication:
31.07.2024 Bulletin 2024/31

(21) Application number: 22872210.4

(22) Date of filing: 26.09.2022

(51) International Patent Classification (IPC):
*G06V 20/69* (2022.01)

(52) Cooperative Patent Classification (CPC):
G01L 5/00; G06N 3/0464; G06N 3/08; G06N 20/10;
G06V 10/764; G06V 10/82; G06V 20/69;
G06V 30/19

(86) International application number:
PCT/CN2022/121340

(87) International publication number:
WO 2023/046167 (30.03.2023 Gazette 2023/13)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 26.09.2021 CN 202111127053

(71) Applicant: Ruixin Fuzhou Technology Co., Ltd
Fuzhou, Fujian 350100 (CN)

(72) Inventor: LIN, Zhe
Fuzhou, Fujian 350100 (CN)

(74) Representative: Garavelli, Paolo
A.BRE.MAR. S.R.L.
Consulenza in Proprietà Industriale
Via Servais 27
10146 Torino (IT)

(54) **CELL RECOGNITION METHOD, APPARATUS, AND SYSTEM**

(57) In order to solve the problem of cell recognition, the inventor provides a cell recognition method, comprising the following steps: obtaining cell information, the cell information comprising cell traction force information of a certain point in a cell obtained on the basis of a cell mechanical sensor, and the cell traction force information comprising the magnitude of a cell traction force at the point; preprocessing the cell information to form structured cell information, the structured cell information comprising the number of cells, the number of cell features, and feature information of respective cell features, and using the structured cell information as input data, establishing a cell feature model by means of supervised, unsupervised, or semi-supervised machine learning, and applying the cell feature model to the classification or clustering of cells of an unknown type or unknown state. The inventor also provides a cell recognition apparatus and cell recognition system implementing the technical solution, which have a high throughput and a high resolution, and are capable of real-time measurement and analysis of living single cells.

FIG. 3

**Description**

FIELD OF THE INVENTION

The present application relates to the field of cell analysis and identification, particularly to a method, device, and system for cell identification.

BACKGROUND OF THE INVENTION

Current prevalent technologies for quantitative analysis at the single cell level include single-cell sequencing technologies (scRNA-seq), characterized by quantitative analysis of the transcriptome of individual cells. However, a limitation of this approach is that it essentially takes a snapshot of the single cell, employing an invasive and destructive method, which does not allow for real-time monitoring of the same cell. Other methods, such as immunofluorescence, require staining of the cells, which inevitably affects or damages the cells, with the process being costly and complex.

SUMMARY OF THE INVENTION

Therefore, it is necessary to provide a high-throughput, high-resolution, and real-time analysis of live single cells for cell identification, which further aids in scientific research, drug development, and clinical applications.

In order to achieve the above objects, the present application providing a method for cell identification, comprising the following steps:

Acquiring cell information, the cell information comprising cellular traction force information at a point within a cell obtained via a cellular mechanical sensor, the cellular traction force information comprising the magnitude of the cellular traction force at the point; preprocessing the acquired cell information to generate structured cell information, the structured cell information comprising the number of cells, the number of cell features, and feature information for each cell feature; and inputting the structured cell information into a machine learning model established through supervised, unsupervised, or semi-supervised learning, and applying the machine learning model to classify or cluster cells of unknown types or states.

Furthermore, the cellular traction force information also includes the direction of the cellular traction force at this point.

Furthermore, the cellular traction force information also includes the changes of the magnitude or direction of the cellular traction force at the point within a certain time interval.

Furthermore, the cell information also includes cell morphology information.

Furthermore, the cell information is obtained by performing cell confining operations on the cell.

The present application also provides a cell identification device, including: an information acquisition unit configured to acquire cell information, the cell information comprising cellular traction force information at a point within a cell obtained via a cellular mechanical sensor, the cellular traction force information comprising the magnitude of the cellular traction force at that point; a preprocessing unit configured to preprocess the cell information to generate structured cell information, the structured cell information comprising the number of cells, the number of cell features, and information for each cell feature; a learning unit configured to use the structured cell information as input data for establishing a cell feature model via supervised, unsupervised, or semi-supervised learning; and an identification unit configured to apply the cell feature model to classify or cluster cells of unknown types or states.

Furthermore, the cellular traction force information also includes the direction of the cellular traction force at the point.

Furthermore, the cellular traction force information also includes the change of the magnitude or direction of the cellular traction force at the point within a certain time interval.

Furthermore, the cell information also includes cell morphology information.

Furthermore, the cell information is obtained by performing cell confining operations on the cell.

The present application also provides a cell identification system, comprising: a cellular mechanical sensor and the above cell identification device.

Furthermore, the cellular mechanical sensor includes a nano micropillar array, or a cellular traction force detection device with a light-reflective layer on the micropillars .

Furthermore, a device for detecting cellular mechanical force, including: a base; and an micropillar array of multiple micropillars located on the base, the micropillars being deformable in respond to a cellular mechanical force, and the top or the upper portion of the pillar surface of the micropillars having a light-reflective layer.

Furthermore, the cell identification system further includes a device for acquiring cell morphology information.

Furthermore, the device for acquiring cell morphology information thereof includes a microscopic camera or a micro camera.

Furthermore, the cell identification system thereof further includes a cell confining device for performing cell confining operations on the cell.

The present application also provides a method for detecting cell state comprising: obtaining cellular traction force information using the above method for identification, the above device for identifying cells, or the above system for identifying cells, and analyzing the cell state based on the cellular traction force information; the cell state includes cell adhesion, cell viability, cell differentiation/activation, cell proliferation, and/or cell migration.

Furthermore, in any of the above schemes, the cells may be individual cells or multicellular aggregates formed by two or more cells. The present application is not limited to the various forms formed by individual cells or two or more cells.

Different from the existing technology, the above-mentioned technical solution has the following advantages: the present application uses a cellular mechanical sensor to obtain cell mechanical information for cell identification, and the cell identification includes not only the type of the cell, but also the state of the cell; the technical solution in the present application has non-invasive effects on living cells, with significant advantages of real-time, high-throughput, and high-resolution; based on the measurement of the cellular traction force of each single cell, the technical solution in the present application can identify different types of cells, and can be further used to detect the influence of cell force on chemotherapy drugs, even cell sorting, which has great application value in biomedicine and medical treatment. Furthermore, the technology presented in this application enables the detection of cellular traction force from multicellular aggregates, such as tumor spheroids and organoids. This capability makes it suitable for applications like drug screening and situations that demand the characterization of multicellular aggregates in regenerative medicine, gene editing, precision medicine, organ development, and disease modeling scenarios.

## BRIEF DESCRIPTION OF THE DRAWINGS

FIG. 1 shows a schematic diagram for scalar processing of displacement information at a specific point according to the fourth embodiment of the present application.

FIG. 2 shows result diagram A for the identification of unknown cells or unknown cellular phenotypes using the established cell feature model, as extended in the fifth embodiment of the present application.

FIG. 3 shows result diagram B for the identification of unknown cells or unknown cellular phenotypes using the established cell feature model, as extended in the fifth embodiment of the present application.

FIG. 4 shows a schematic structural view of the cell identification device according to the twelfth embodiment of the present application.

FIG. 5 shows a schematic structural view of the cell identification system as described in the eighteenth embodiment of the present application.

FIG. 6 shows a schematic structural view of the cellular traction force detection device as described in the twentieth embodiment of the present application.

FIG. 7 shows a schematic structural view of the cell identification system as described in the twentieth embodiment of the present application.

FIG. 8 includes (a), (b) and (c), (a) shows a schematic structural view of the cell identification system as described in the twentieth embodiment of the present application.

FIG. 8(b) shows an image of the cellular traction force detection device's light reflection signal obtained by the information acquisition unit of the cell identification system described in the twentieth embodiment of the present application.

In FIG. 8(c) shows a visualization effect diagram of mechanical size and distribution processed by the preprocessing unit of the cell identification system as described in the twentieth embodiment of the present application.

FIG. 9 includes (a), (b) and (c), (a) shows a real image of cellular traction force detection device using silicon film as a cell confinement device.

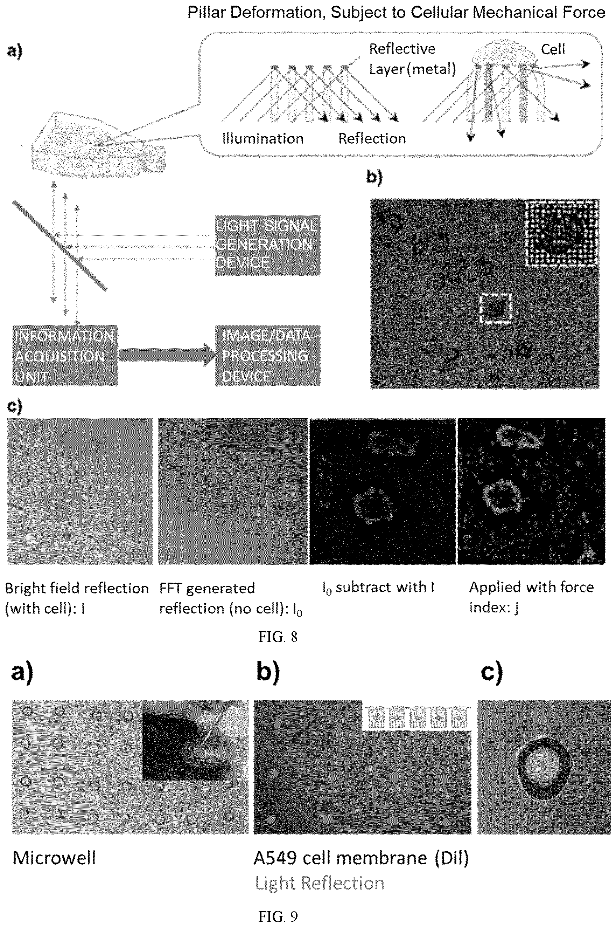

In FIG. 9, (b) shows a fluorescence microscope image of cellular traction force detection device using silicon film as a cell confinement device under light reflection.

In FIG. 9 (c) is an enlargement image of (b) in FIG. 9.

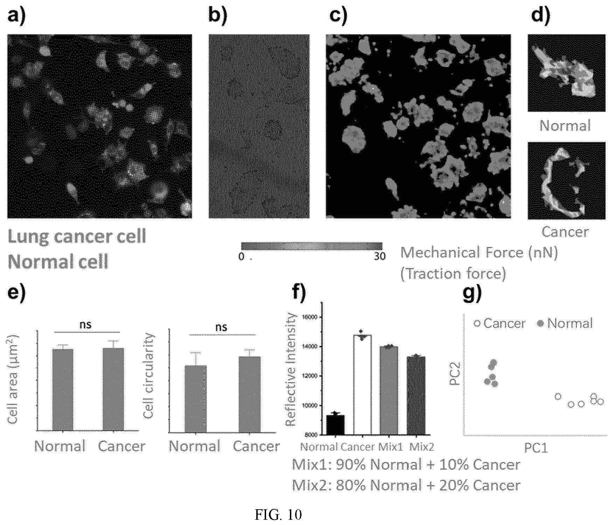

FIG. 10 includes (a), (b) and (c), (d), (e), (f) and (g), (a) shows a fluorescence imaging diagram of a mixed system of healthy cells and lung non-small cell carcinoma cells.

In FIG. 10, (b) shows a distribution diagram of light reflection signals of cellular traction force detection device obtained by the information acquisition unit.

In FIG. 10, (c) shows a visualization effect diagram of mechanical size and distribution processed by preprocessing unit.

In FIG. 10, (d) shows an enlargement diagram of the representative single-cell force distribution of healthy cells and lung non-small cell carcinoma cells as shown in (c) in FIG. 10.

In FIG. 10, (e) shows a comparative diagram of cell morphology between healthy cells and lung non-small cell carcinoma cells.

In FIG. 10, (f) shows a comparative diagram of reflection signal intensities of healthy cells, lung non-small cell carcinoma cells, and a mixture of these two cell types in different proportions.

In FIG. 10, (g) shows a cluster analysis diagram based on structured cell information processed from (c) in FIG. 10.

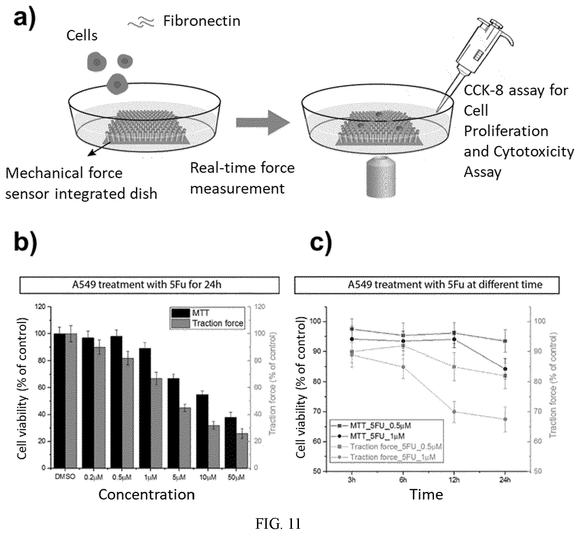

FIG. 11 includes (a), (b) and (c), (a) shows a schematic diagram of operational procedure for a method for detecting cell vitality.

In FIG. 11, (b) shows a comparative diagram of cell vitality as determined by the MTT assay and reflected by cellular traction force in A549 cells following treatment with various doses of 5-FU for 24 hours.

In FIG. 11, (c) shows a comparative diagram of cell vitality determined by the MTT assay and reflected by cellular traction force in A549 cells after treatment with various doses of 5-FU for different time.,

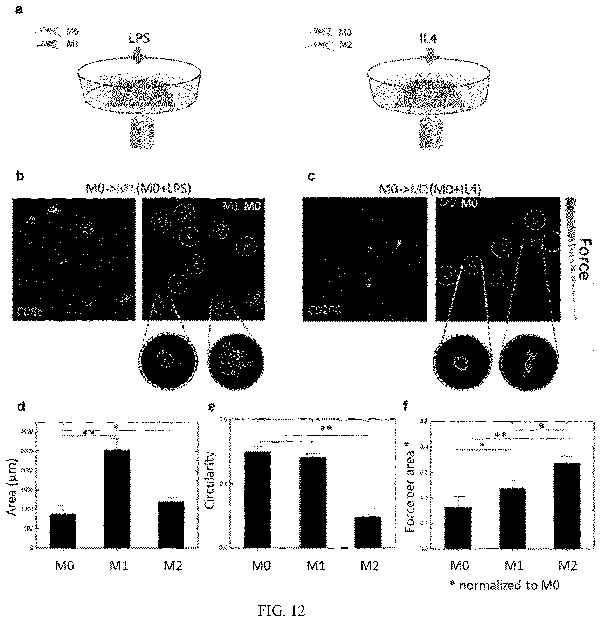

FIG. 12 includes (a), (b), (c), (d), (e) and (f), (a) shows a process diagram for a method for detecting cell state.

In FIG. 12, (b) shows a fluorescence microscopy image of M0 macrophages distinct into M1 state.

In FIG. 12, (c) shows a fluorescence microscopy image of M0 macrophages distinct into M2 state.

In FIG. 12, (d) shows a comparative diagram of cell adhesion area for M0 macrophages, M1 state, and M2 state.

In FIG. 12, (e) shows a comparative diagram of cell roundness for M0 macrophages, M1 state, and M2 state.

In FIG. 12, (f) shows a comparative diagram of cellular traction force for M0 macrophages, M1 state, and M2 state.

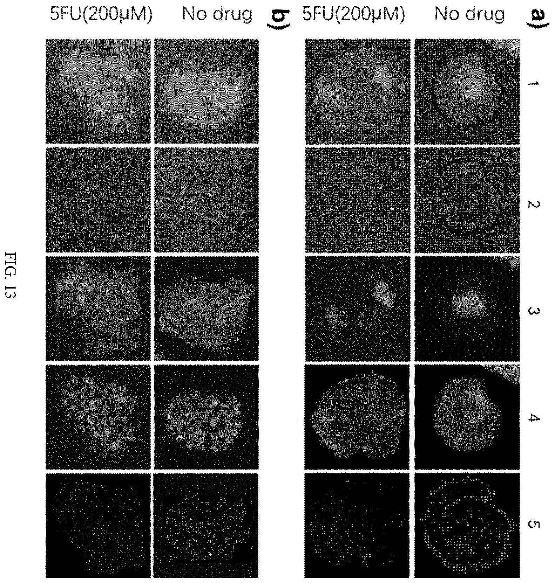

FIG. 13 includes (a), and (b), (a) shows characterization diagrams for a tumor cell aggregate exhibiting a first morphology before and after treatment with/without 5-FU; from left to right: a composite image of cell membrane fluorescence and reflection signal (1), light reflection signal (2), nucleus (3), cell membrane (4), and cell force visualization image processed by optical image analysis software (ImageJ) (5).

In FIG. 13, (b) shows characterization diagrams for a tumor cell aggregate exhibiting a second morphology before and after treatment with/without 5-FU; from left to right: a composite image of cell membrane fluorescence and reflection signal (1), light reflection signal (2), nucleus (3), cell membrane (4), and cell force visualization image processed by optical image analysis software (ImageJ) (5).

Labels in the drawing: 1, information acquisition unit; 2, preprocessing unit; 3, learning unit; 4, identification unit; 10, cellular mechanical sensor (cellular traction force detection device); 101, base; 102, light signal generation device; 103, micropillars; 104, spectroscope; 1031, light-reflective layer; 20, cell identification device; 201, image/data processing device; 30, cell morphology information acquisition device; 40, cell confinement device;

DETAILED DESCRIPTION OF PREFERRED EMBODIMENTS

To provide a detailed description of technical content, constructional features, objectives, and effects of the technical solutions, the following explanations are provided in conjunction with specific embodiments and accompanying drawings.

First Embodiment

A method for cell identification (only based on the magnitude of cellular traction forces, unlabeled) includes the following steps:

S1: acquiring cell information, where the cell information is derived from the magnitude of cellular traction force at specific points within the cells, as measured by a cellular mechanical sensor. This involves collecting cell information from multiple cells, including the acquiring cellular traction force magnitude data from multiple points within each cell, thereby obtaining a dataset of the multi-point cellular traction force magnitudes across multiple cells.
S2: preprocessing the acquired cellular traction force magnitude data to form structured cell information. The structured information includes the number of cells, the number of cellular features, and the feature information for each cellular feature. The structured cell information can be viewed as a two-dimensional feature matrix $M_{N \times P}$ (Feature matrix), wherein N represents the number of cells and P represents the number of cellular features, with P=1 indicating the cellular feature as the magnitude of cellular traction force.
S3: using the structured cell information as input data, employing unsupervised machine learning to establish a cell feature model, and applying the model for clustering cells with unknown types or states.

In similar embodiments to the first, further optimization or improvement can be achieved by processing the multiple-point cellular traction force magnitude data for an individual cell to calculate additional dimensions of information, such as the average cellular traction force magnitude per unit area or the distribution of cellular traction force magnitudes within the cell. These new cellular features can be incorporated into the two-dimensional feature matrix described in step S2, thereby expanding the content of P. Subsequently, machine learning is used to determine which features can more effectively distinguish among cells of different types or states.

Second Embodiment

A method for cell identification (only based on the magnitude of cellular traction force, with labeled) includes the following steps:

S1: acquiring cell information for several kinds of cells with known cell types or states, the information includes the magnitude of cellular traction force at specific points within the cells, as measured by a cellular mechanical sensor. This involves collecting information from various cell types, including acquiring cellular traction force magnitude data from multiple points within each cell, thereby obtaining cellular traction force magnitude data across multiple cells.
S2: preprocessing the acquired cellular traction force magnitude data to form structured cell information. The structured cell information includes the number of cells, the number of cellular features, and the feature information for each cellular feature. At this stage, the structured information can be viewed as a $M_{N \times P}$ two-dimensional feature matrix, wherein N represents the number of cells and P represents the number of cellular features, with P=1 indicating the cellular feature as the magnitude of cellular traction force.
S3: using the structured cell information as input data, employing supervised machine learning to establish a cell feature model and to train the model with a large dataset of structured cell information. For example, the present embodiment utilizes the Random Forest (RF) algorithm for significant feature extraction and model parameter estimation, and then the model is applied to new cells to estimate the labels thereof, thus classifying unknown types or states of cells (identification in this context should be understood in a broad sense, including both "classification" and "clustered" cells that may have the same or similar properties and potentially belong to the same or similar types or states together, although the specific cell state or type is unknown). In other implementation manners, machine learning algorithm slide as such as support vector machine (Support Vector Machine, SVM) or deep learning may also be adopted to complete the corresponding model building and training and learning work.

In some other implementations similar to the present embodiment, optimization or improvement can be carried out in the following manner: For an individual cell, further information processing is performed on the information on the magnitude of the multi-point cellular traction force obtained by the individual cell, such as calculating the average value of the cellular traction force per unit area and the distribution of the cellular traction force in the cell. Such information can be used as a new cell feature and added to the two-dimensional feature matrix described in step S2, that is, to expand the content of P, then through subsequent machine learning to know which feature can better distinguish cells of different types or states.

Third Embodiment

A method for cell identification (only based on the magnitude of cellular traction forces, with partial labeled and partial unlabeled) includes the following steps:

S1: acquiring cell information from multiple cells, including both cells of known types or states and cells of unknown types or states, with information based on the magnitude of cellular traction force at specific points within the cells as measured by a cellular mechanical sensor.

S2: preprocessing the acquired cellular traction force magnitude data to form structured cell information. The structured cell information includes the number of cells, the number of cellular features, and the feature information for each cellular feature. At this stage, the structured information can be viewed as a $M_{N \times P}$ two-dimensional feature matrix, wherein N represents the number of cells and P represents the number of cellular features, with P=1 indicating the cellular feature as the magnitude of cellular traction force.

S3: using the structured cell information as input data, employing semi-supervised machine learning to establish a cell feature model and to train the model with a large dataset of structured cell information (including both labeled and unlabeled cells) for classification/clustering of the cells of unknown types or states.

In some other implementations similar to the present embodiment, optimization or improvement can be carried out in the following manner: For an individual cell, further information processing is performed on the information on the magnitude of the multi-point cellular traction force of the individual cell, such as calculating the average value of the cellular traction force per unit area and the distribution of the cellular traction force in the cell. Such information can be used as a new cell feature and added to the two-dimensional feature matrix described in step S2, that is, to expand the content of P, then through subsequent machine learning to know which feature can better distinguish cells of different types or states.

Fourth Embodiment

A method for cell identification (based on the magnitude and direction of cellular traction forces, without labeled) includes the following steps:

S1, collecting cell information, the cell information refers to the magnitude and direction of cellular traction forces at specific points within cells obtained by using cellular mechanical sensors (nano micropillars sensors in the present embodiment). This involves collecting cell information on multiple cells, including data on the magnitude and direction of cellular traction forces at various points within each cell, thereby acquiring data on cellular traction force magnitude and direction at multiple points across multiple cells;

S2, Preprocessing the collected information on cellular traction force magnitude and direction to form structured cell information. The structured cell information includes the number of cells, the number of cell features, and information for each cell feature. At this stage, the structured cell information can be viewed as a $M_{N \times P}$ two-dimensional feature matrix, wherein N represents the number of cells and P represents the number of cell features, with P=2 here, indicating the cell features are cellular traction force magnitude and direction;

S3, using the aforementioned structured cell information as input data to establish a cell feature model via unsupervised machine learning, and applying the cell feature model for clustering cells of unknown types or states.

Specifically, the S2 step in the present embodiment processes cell information approximately as follows: Suppose cellular traction force vector data (magnitude and direction) are collected at n points within a cell. These points are denoted as: $(i, i \in \{1,2, ... n\})$, corresponding to two-dimensional coordinates: $(x_{t_0 i}, y_{t_0 i})$ and $(x_{t_n i}, y_{t_n i})$, wherein and $t_n$ represent the initial and displaced positions of the nano micropillars, respectively. Thus, $(x_{t_n i} - x_{t_0 i}, y_{t_n i} - y_{t_0 i})$ denotes the direction of force at each coordinate point. Additionally, each coordinate point should have a scalar information, i.e., the magnitude of the force *d*. Hence, it's possible to estimate the cell's axis direction and central point coordinates based on the existing data, organizing each cell as a vector of equal length. For example, based on the points within each cell, the central point can be calculated as: $\frac{1}{n}\sum_i(x_i, y_i)$ . The cell axis is determined by finding the two points $(x_1, y_1)$ and $(x_2, y_2)$ that are furthest apart, with the cell axis calculated using the following formula:

$$\beta = \frac{y_2 - y_1}{x_2 - x_1}, \alpha = y_2 - \beta x_2, y = \alpha + \beta x$$

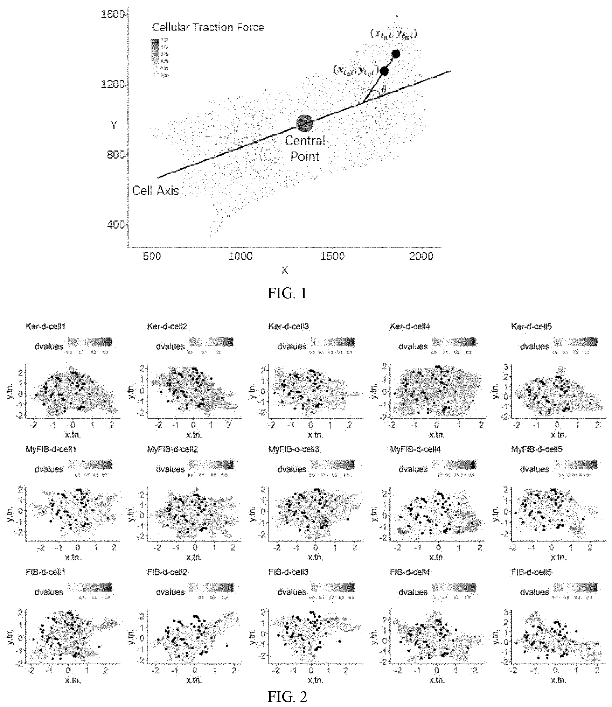

Referring to FIG. 1, which illustrates a schematic diagram for scalar processing of displacement information at a specific point according to the fourth embodiment of the present application. Each point represents a location, with the color depth of the points transitioning from light to dark to signify the force magnitude increasing from small to large. After determining the cell axis for each cell, further quantification of each point is possible: calculating the angle between each point's displacement vector and the cell axis. Each point can then be combined with the magnitude of the force *d* (scalar) as a weight, thereby processing each point into a scalar $s_i = \theta_i * d_i$. Consequently, the cell information for each cell can be organized into a vector

$$z = (s_1, \dots s_n)$$

.

In other embodiments similar to the present embodiment, optimizations or improvements can be made as follows: For individual cell, further processing of the collected data on cellular traction force magnitude or direction, such as calculating the average magnitude of cellular traction force per unit area, distribution of cellular traction force magnitude within the cell and distribution of cellular traction force vectors within the cell, etc., can serve as new cell features to be added to the two-dimensional feature matrix described in step S2, thereby expanding the content of P. Subsequent machine learning can then determine which features better distinguish cells of different types or states.

Fifth Embodiment

A Method for Cell Identification (Magnitude and Direction of Cellular Traction Forces, Labeled)

S1, collecting cell information for several kinds of cells with known cell types or states, where the cell information refers to the magnitude and direction of cellular traction force at specific points within cells, obtained by using cellular mechanical sensors. This includes using cellular mechanical sensors to collect information on multiple cells, including data collection on the magnitude of cellular traction force at various points within each cell, thereby acquiring data on cellular traction force magnitude at multiple points across multiple cells.
S2, preprocessing the collected information on cellular traction force magnitude to form structured cell information. The structured cell information includes the number of cells, the number of cell features, and information for each cell feature. At this stage, the structured cell information can be viewed as a $M_{N \times P}$ two-dimensional feature matrix, wherein N represents the number of cells, and P represents the number of cell features, with P=2 here, indicating the cell features are cellular traction force magnitude and direction.
S3, using the aforementioned structured cell information as input data to establish a cell feature model via supervised machine learning and using the structured cell information of a large number of cells to train the cell feature model. The model is then applied to classify cells of unknown types or states. For example, the present embodiment utilizes the Random Forest (RF) algorithm for significant feature extraction and model parameter estimation, then applies it to new cells to predict the labels thereof, thereby classifying cells of unknown types or states. Other embodiments might employ machine learning algorithms/methodologies such as Support Vector Machine (SVM) or deep learning for corresponding model building and training.

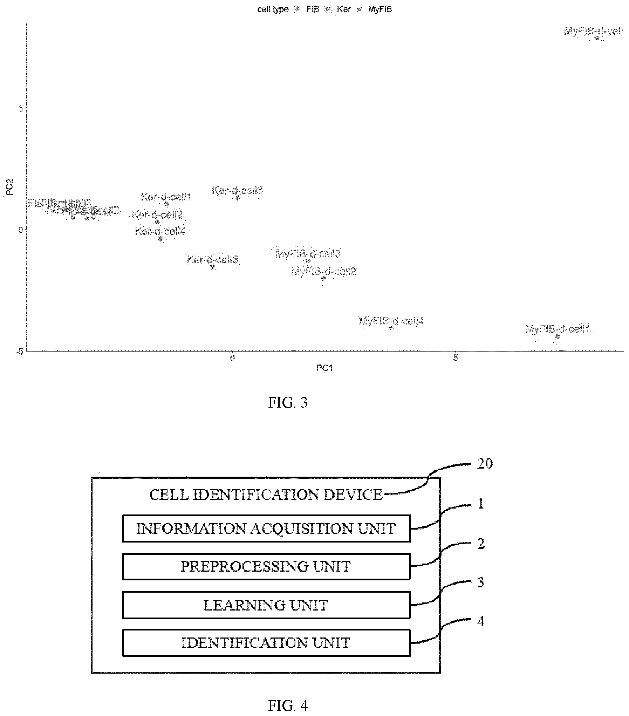

Refer to FIG.s 2 and 3, which show the results of applying the established cell feature model to the identification of unknown cells or cell phenotypes in an extended implementation of the fifth embodiment of the present application. The result diagram A (FIG. 2) shows different rows representing different cell types, and different columns representing different samples. The black dots represent the top 50 significant features extracted using the Random Forest algorithm, also referred to as significant points. These points denote specific positions within a cell, from which varying cellular traction force information is obtained. The result diagram B (FIG. 3) displays the distinctive differentiation effects of using the top 50 significant features (significant points) on three different cell types. The significant features learned from labeled data enable dimensionality reduction and visualization of the data. Subsequent cluster algorithms, based on reduced dimensionality data, can be used for cell classification and identification.

In other embodiments similar to the present embodiment, optimizations or improvements can be made as follows: For individual cells, further processing of the collected data on cellular traction force magnitude or direction, such as calculating the average magnitude of cellular traction force per unit area, the distribution of cellular traction force magnitude within the cell and the distribution of cellular traction force vectors within the cell; etc., can serve as new cell features to be added to the two-dimensional feature matrix described in step S2, thereby expanding the content of P. Subsequent machine learning can then determine which features better distinguish cells of different types or states.

Sixth Embodiment

A method for cell identification (magnitude and direction of cellular traction force, with data partially labeled and partially unlabeled), involving the following steps:

S1, collecting cell information from multiple cells, some of which are of several kinds of cells with known cell types or states, while the rest are of cells with unknown cell types or states; the cell information refers to the magnitude of cellular traction forces at specific points within cells, obtained using cellular mechanical sensors. This involves using cellular mechanical sensors to collect information on various cells, including data collection on the magnitude of cellular traction forces at various points within each cell, thereby acquiring data on cellular traction force magnitude at multiple points across multiple cells.

S2, preprocessing the collected information on cellular traction force magnitude to form structured cell information. The structured cell information includes the number of cells, the number of cell features, and information for each cell feature. At this stage, the structured cell information can be viewed as a $M_{N \times P}$ two-dimensional feature matrix, wherein N represents the number of cells, and P represents the number of cell features, with P=2 here, indicating the cell features are cellular traction force magnitude and direction.

S3, using the aforementioned structured cell information as input data to establish a cell feature model via semi-supervised machine learning and using the structured cell information of large number of cells to train the cell feature model, then applying it to classify cells of unknown types or states.

In other embodiments similar to the present embodiment, optimizations or improvements can be made as follows: For individual cells, further processing of the collected data on cellular traction force magnitude or direction, such as calculating the average magnitude of cellular traction force per unit area, the distribution of cellular traction force magnitude within the cell and the distribution of cellular traction force vectors within the cell, etc., can serve as new cell features to be added to the two-dimensional feature matrix described in step S2, thereby expanding the content of P. Subsequent machine learning can then determine which features better distinguish cells of different types or states .

Seventh Embodiment

A method for cell identification (instantaneous values of cellular traction force vectors, and changes thereof within a certain time interval, unlabeled), includes the following steps:

S1, collecting cell information, where the cell information includes the instantaneous values of cellular traction force vectors at specific points within cells and the changes in these cellular traction force vectors within a certain time interval, obtained using cellular mechanical sensors. This involves using cellular mechanical sensors to collect information on multiple cells, including data collection on the magnitude and direction of cellular traction force at various points within each cell, thereby acquiring data on cellular traction force magnitude and direction at multiple points across multiple cells.

S2, preprocessing the collected information on cellular traction force magnitude and direction to form structured cell information. The structured cell information includes the number of cells, the number of cell features, and information for each cell feature. At this stage, the structured cell information can be viewed as a $M_{N \times P}$ two-dimensional feature matrix, where N represents the number of cells, and P represents the number of cell features.

S3, using the aforementioned structured cell information as input data to establish a cell feature model via unsupervised machine learning, and applying the cell feature model for clustering cells of unknown types or states.

In the present embodiment, since the data includes not only the instantaneous values of the cellular traction force vectors at specific points within the cell but also the changes thereof within a certain time interval, the mechanical data for individual cells can effectively be analogized to images (instantaneous values) or videos (changes over time). If the array of points covered by the current cell is likened to pixels in an image, and the information recorded at each point (force magnitude, direction, etc.) is analogized to the colors corresponding to pixels, subsequent machine learning can draw on algorithms used in the image and video data processing domains. Specifically, this may involve employing machine learning algorithms widely used in image recognition, more precisely, deep learning, such as Convolutional Neural Networks (CNN), for data modeling and analysis.

Eighth Embodiment

A method for cell identification (instantaneous values of cellular traction force vectors, and changes thereof within a certain time interval labeled), includes the following steps:

S1: acquiring cell information for several kinds of cells with known cell types or states, where the information includes instantaneous values of cellular traction force vectors at specific points within cells and the changes of these vectors within a certain time interval, using a cellular mechanical sensor. This involves collecting cell information across multiple cells, including the acquiring cellular traction force magnitude and direction data from multiple points within each cell, thereby gathering data on the magnitude and direction of cellular traction force at multiple points across multiple cells.

S2: preprocessing the acquired data on the magnitude and direction of cellular traction force to form structured cell information. The structured information includes the number of cells, the number of cellular features, and information for each feature. At this stage, the structured cell information can be viewed as a $M_{N \times P}$ two-dimensional feature matrix (Feature matrix), where N represents the number of cells, and P represents the number of cellular features.

S3: using the structured cell information as input data, employing supervised machine learning to establish a cell feature model. The model is trained with a substantial dataset of structured cell information and then applied to classify unknown types or states of cells. For example, the present embodiment adopts the Random Forest (RF) algorithm for significant feature extraction and model parameter estimation, subsequently applied to new cells to estimate the labels thereof, thus classifying cells of unknown types or states. Alternative embodiments may employ Support Vector Machine (SVM) or deep learning algorithms for model establishment and training.

The present embodiment's methodology, capturing not only the instantaneous values of cellular traction force vectors at specific points but also the changes thereof within a certain time interval, and then the mechanical data of a single cell can be analogized to images (instantaneous values) or videos (changes over time). If the array of points covered by the current cell is likened to pixels in an image, and the information recorded at each point (force magnitude, direction, etc.) is analogized to the colors corresponding to pixels, subsequent machine learning can draw on algorithms used in the image and video data processing domains. Specifically, this may involve employing machine learning algorithms widely used in image recognition, more precisely, deep learning, such as Convolutional Neural Networks (CNN), for data modeling and analysis.

Ninth Embodiment

A method for cell identification incorporating instantaneous values and changes within a certain time interval of cellular traction force vectors, with partially labeled and partially unlabeled data, includes the steps of:

S1: acquiring cell information from several kinds of cells with known cell types or states, where the information includes instantaneous values of cellular traction force vectors at specific points within cells and the changes of these vectors within a certain time interval, using a cellular mechanical sensor. This involves collecting cell information across multiple cells, including the acquiring cellular traction force magnitude and direction data from multiple points within each cell, and the change information of cellular traction force vectors within a certain time intervals.

S2: preprocessing the acquired data on the magnitude and direction of cellular traction force to form structured cell information. This structured information includes the number of cells, the number of cellular features, and information for each feature. At this stage, the structured cell information can be viewed as a $M_{N \times P}$ two-dimensional feature matrix, wherein N represents the number of cells, and P represents the number of cellular features.

S3: using the structured cell information as input data, employing semi-supervised machine learning to establish a cell feature model. The model is trained with a substantial dataset of structured cell information and then applied to classify/cluster cells of unknown types or states.

In the present embodiment, since what is acquired is not only the instantaneous value of the cellular traction force vector at a certain point in the cell, but also the changes thereof within a certain time interval, the mechanical data for individual cells can effectively be analogized to images (instantaneous values) or videos (changes over time). If the array of points covered by the current cell is likened to pixels in an image, and the information recorded at each point (force magnitude, direction, etc.) is analogized to the colors corresponding to pixels, subsequent machine learning can draw on algorithms used in the image and video data processing domains. Specifically, this may involve employing machine learning algorithms widely used in image recognition, more precisely, deep learning, such as Convolutional Neural Networks (CNN), for data modeling and analysis.

Tenth Embodiment

A cell identification method differs from the first to ninth embodiments by including cell morphological information in the cell data. The morphological information is captured using a microscope camera or a microscope video camera, particularly when continuous data collection within a certain time interval is required. During the data preprocessing step, cell features now also encompass cell morphological information or further information derived from processing or analyzing the morphological data. This includes one or several of the following: cell size, cell shape, nucleus size, nucleus shape, and cell color.

Eleventh Embodiment

A cell identification method, distinct from the first to tenth embodiments, involves collecting cell data under conditions where cells are physically constrained. Methods for physically constraining cell morphology include, for example, constructing confining walls around several cellular mechanical sensors to enclose a defined area (surface area). The height of the confining walls exceeds that of the enclosed cellular mechanical sensors, acting as a cell confinement device. Given the defined spatial area, only cells of compatible sizes can come into the defined spatial area and contact with the cellular mechanical sensors, typically accommodating a single cell within the confines thereof. In special circumstances, these confining walls can also exert a compressive effect, making the enclosed cells conform to some extent to the cross-sectional shape of the confinement, thereby achieving a certain degree of cell morphology confinement.

Compared to the cell identification methods that do not constrain cells, imposing confinements (whether in number or morphology) can reduce cell-to-cell contact, ensuring most cells are in a single-cell state; also can simplify the analysis by reducing the dimensionality related to cell size and shape, that is, the number of the cell features is reduced; and specific cell shapes can influence the differentiation state of cells, such as confining immune cells to an M1 state in certain scenarios, yielding more valuable results.

Of course, without morphological constraints, cells remain in natural state thereof, minimizing external interventions' impact on cells. Moreover, cell edge detection to determine cell morphology and polarity, aiding cell identification and prediction, is only feasible without morphological constraints. In summary, the cell confinement strategy described in the present embodiment offers unique advantages in specific technical scenarios.

Twelfth Embodiment

Referring to FIG. 4, the present embodiment introduces a cell identification device comprising an information acquisition unit 1, a preprocessing unit 2, a learning unit 3, and an identification unit 4;

The information acquisition unit 1 is used for gathering cell data, including the magnitude of cellular traction force at specific points within cells as measured by a cellular mechanical sensor.

The preprocessing unit 2 preprocesses the cell data to create structured cell information, which includes the number of cells, the number of cellular features, and the information for each feature.

The learning unit 3 uses the structured cell information as input data to develop a cell feature model through supervised, unsupervised, or semi-supervised machine learning techniques.

The identification unit 4 applies the cell feature model to classify or cluster cells of unknown types or states.

The cell identification device described in the present embodiment can implement the cell identification methods outlined in the first to third embodiments.

Thirteenth Embodiment

A cell identification device, distinct from the twelfth embodiment, the information acquisition unit 1 thereof also captures the direction of cellular traction force. This cell identification device is capable of implementing the cell identification methods as described in the fourth to sixth embodiments.

Fourteenth Embodiment

A cell identification device, distinct from the twelfth and thirteenth embodiments, the cellular traction force information obtained by the information acquisition unit 1 comprises the magnitude or the direction of cellular traction force within a certain time interval. The present embodiment's cell identification device facilitates the implementation of the cell identification methods as outlined in the seventh to ninth embodiments.

Fifteenth Embodiment

A cell identification device, distinct from the twelfth to fourteenth embodiments, the cellular traction force information obtained by the information acquisition unit 1 also includes cell morphological information. The present embodiment's cell identification device supports the implementation of the cell identification method described in the tenth embodiment.

Sixteenth Embodiment

A cell identification device, distinct from the twelfth to fifteenth embodiments, the cell data is collected under cellular confinement operations. Methods for physically constraining cell morphology include, for example, constructing confining walls around several cellular mechanical sensors to enclose a defined area (surface area). The height of the confining walls exceeds that of the enclosed cellular mechanical sensors, acting as a cell confinement device. Given the defined spatial area, only cells of compatible sizes can come into contact with the cellular mechanical sensors, typically accommodating a single cell within the confines thereof. In special circumstances, these confining walls can also exert a compressive effect, making the enclosed cells conform to some extent to the cross-sectional shape of the confinement, thereby achieving a certain degree of cell morphology confinement.

Compared to the cell identification methods that do not constrain cells, imposing confinements (whether in number or morphology) can reduce cell-to-cell contact, ensuring most cells are in a single-cell state; also can simplify the analysis by reducing the dimensionality related to cell size and shape, that is, the number of the cell features is reduced; and specific cell shapes can influence the differentiation state of cells, such as confining immune cells to an M1 state in certain scenarios, yielding more valuable results.

Of course, without morphological constraints, cells remain in natural state thereof, minimizing external interventions' impact on cells. Moreover, cell edge detection to determine cell morphology and polarity, aiding cell identification and prediction, is only feasible without morphological constraints. In summary, the cell confinement strategy described in the present embodiment offers unique advantages in specific technical scenarios.

The cell identification device described in the present embodiment can be used to realize the technical solution of the cell identification method described in the eleventh embodiment.

Seventeenth Embodiment

A cell identification system comprises a cellular mechanical sensor 10 and a cell identification device 20. The cell identification device corresponds to those described in the twelfth to sixteenth embodiments, designed to implement the cell identification methods detailed in the first to ninth embodiments.

In the present embodiment, the cellular mechanical sensor 10 is a nano micropillar array, and other implementations may use any device capable of capturing cellular traction force information as the cellular mechanical sensor.

Eighteenth Embodiment

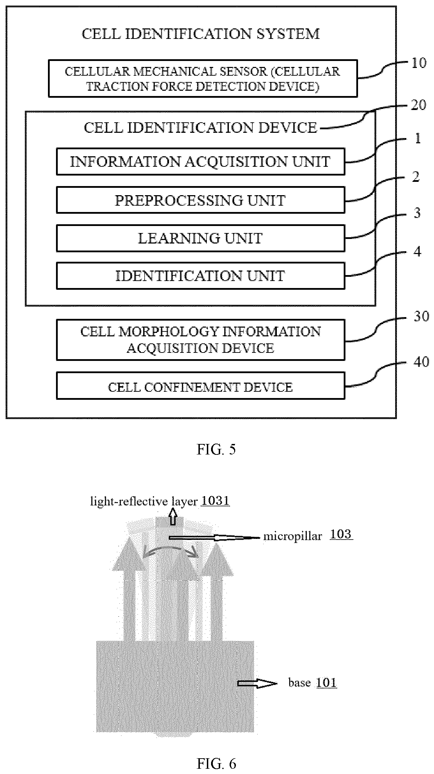

Referencing FIG. 5, the present embodiment introduces a cell identification system that is different from the seventeenth embodiment by incorporating a cell morphology information acquisition device 30, designed to capture cell morphology information. In the present embodiment, the cell morphology information acquisition device 30 is a microscope camera. In other implementations, device 30 could also be a video microscope or any other device capable of capturing cell morphology information. The cell identification system is capable of implementing the cell identification method as outlined in the tenth embodiment.

Nineteenth Embodiment

A cell identification system, distinct from the seventeenth and eighteenth embodiments, includes a cell confinement device 40 for conducting cellular confinement operations. In the present embodiment, the cell confinement device 40 is structured as follows: confining walls are constructed around several cellular mechanical sensors to enclose a specific area (surface area), with the walls' height exceeding that of the cellular mechanical sensors within, thus serving as a cell confinement device. The setup defines a spatial area such that only cells of a suitable size can fall into it and make contact with the cellular mechanical sensors, typically accommodating one cell. In certain scenarios, these confining walls can also exert a compressive effect, causing the enclosed cells to conform to the shape of the cross-sectional area of the confinement, thus achieving a degree of cellular morphology confinement. The cell identification system can implement the cell identification method described in the eleventh embodiment.

Twentieth Embodiment

A cell identification system, distinct from the seventeenth, eighteenth, and nineteenth embodiments, features a cellular mechanical sensor 10 equipped with a light-reflective layer on micropillars for detecting cellular traction forces. Alternative embodiments may employ any device capable of capturing cellular traction force information as the cellular mechanical sensor.

Specifically, referring to FIG. 6 for a schematic structural view of the cellular traction force detection device. As illustrated, the present embodiment's cellular traction force detection device comprises a transparent base 101 and micropillars 103 positioned on the base 101, which deform under the action of cellular traction force. The tops of micropillars 103 are coated with a light-reflective layer 1031, with a thickness of 5nm (in other embodiments, the thickness of the light-reflective layer 1031 can range 5nm-20nm, depending on the coating material and the selected thickness to ensure light transmission and micropillar stability, and to secure attachment to the micropillars). The micropillars 103 are designed to transmit light, with opposing arrow clusters indicating the direction of incident and reflected light. (Note: The term "coating" used in the present embodiment implies that the light-reflective layer 1031 may be fabricated through a coating process but does not restrict the fabrication method of the light-reflective layer 1031 to coating processes exclusively.)

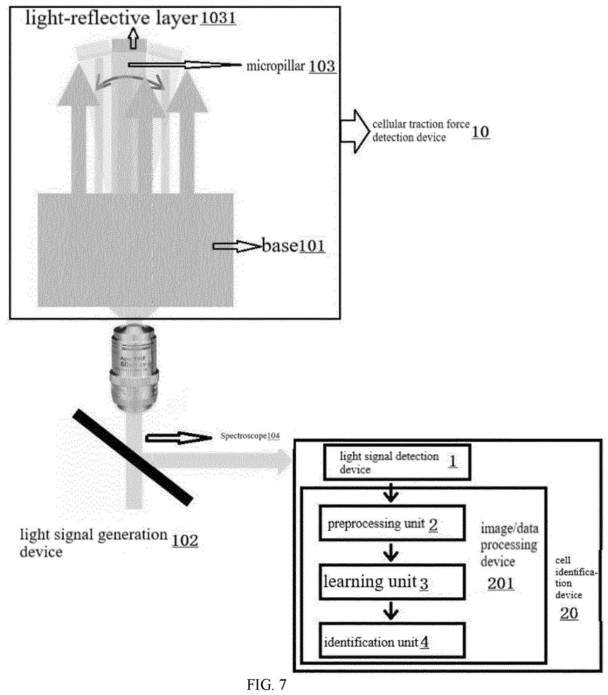

When the cellular traction force detection device 10 of the present embodiment is operational, the number of micropillars 103 will exceed one. Referring to FIG. 7 for a schematic structural view of a cell identification system related to the twenty-first embodiment. Besides the cellular traction force detection device 10 and cell identification device 20 discussed in the present embodiment, the system also includes a light signal generation device 102 with a light source positioned below the base 101 of the cellular traction force detection device 10. The light emitted by the light source illuminates the light-reflective layer 1031 of the micropillars 103 through the incident light path from the transparent base 101 of the cellular traction force detection device 10. The information acquisition unit 1 detects light reflected from the top light-reflective layer 1031 of the micropillars 103, which, after being processed by the beam splitter 104, enters the cell identification device 20. The cell identification device 20's information acquisition unit 1 (light signal detection device) processes the reflected light signal, and the preprocessing unit 2 forms structured cell information. The information includes the number of cells, the number of cell features, and the information for each cell feature. Also, the structured cell information can be viewed as a two-dimensional feature matrix (Feature Matrix), where N is the number of cells and P is the number of cell features, here P=1 or 2, indicating cellular traction force magnitude and/or distribution thereof within the cell. Subsequently, the structured cell information serves as input data for the learning unit 3 to develop a cell feature model through supervised, unsupervised, or semi-supervised learning, and then trained with a large amount of structured cell information, Finally, the identification unit 4 applies the cell feature model to classify or cluster cells of unknown types or states. When micropillars 103 are unforced, they should remain upright, reflecting the detection light maximally, whereas bending under cellular traction force leads to reduced light reflection. Thus, larger cellular traction force result in weaker light reflection signals, allowing for easy deduction of cellular traction force magnitude based on observed light reflection intensity.

In some embodiments of the application, the beam splitter 104 could be a semitransparent or equivalent optical component, primarily aimed at simplifying the optical path design.

The light signal generation device 102 may consist of LEDs, halogen lamps, lasers (e.g., infrared lasers), or other light sources or devices incorporating these light sources, without limitation set by some embodiments of the present application.

Furthermore, the information acquisition unit 1 in the cell identification device could be a microscope, a Charge-Coupled Device (CCD), a Complementary Metal-Oxide-Semiconductor (CMOS) sensor, a Photomultiplier Tube (PMT), a Phototransistor (PT), film, or other equivalent light signal detection components, without restriction set by the present application.

The preprocessing unit, learning unit, and identification unit in the cell identification device may be integrated into an image/data processing device 201, such as optical image analysis software like ImageJ, Matlab, Fluoview, Python, or other equivalent optical image/data analysis components, or a combination of these analysis software, with no specific limitations set by the present application.

The following details the cellular traction force detection and the cell identification analysis process for a cell identification system related to the present embodiment.

Please refer to FIG.s 8a-c, wherein FIG. 8, a illustrates a schematic structural view of the cell identification system; FIG. 8, b displays an image of light reflection signals captured by the information acquisition unit from the cellular traction force detection device; and FIG. 8, c shows a visualization of the magnitude and distribution of mechanics.

As shown in FIG. 8, a, on the cellular traction force detection device, each micropillar is coated with a metal reflective layer on the top thereof, while the sides thereof are coated with an anti-reflective layer. In the absence of cells, the light illuminating the micropillars from below will be fully reflected and then fully captured by the information acquisition unit within the cell identification device (e.g., a CCD camera). However, when cells adhere to the micropillars, the cellular forces generated by cell movement cause the micropillars to tilt, thereby diminishing the reflection signal. After analyzing the light reflection signals, the cellular force intensity can be calculated.

Further, the information acquisition unit (e.g., a CCD camera) collects images of the light reflection signals from the cellular traction force detection device, and enlargement images of local cell adhesion areas (as shown in FIG. 8, b). Subsequently, the preprocessing unit processes the images from FIG. 8, b further, transforming them into a more intuitive visualization of mechanical force (also named as cellular traction force) magnitude and distribution as shown in FIG. 8, c.

The specific processing procedure is as follows: first, based on FIG. 8, b, a clear-field reflection signal image (I, focused on the cell) is obtained. Then, by performing a Fourier transform on the image to filter out high-frequency signals and conducting an inverse Fourier transform, an image of the micropillar reflection signals in an unshifted condition ($I_0$) is calculated. Following this, images I and $I_0$ are further processed to convert the reflection signal image into a more intuitive mechanical force map (subtracting the I signal value from the $I_0$ signal value) and then normalized to obtain a more intuitive visualization of cellular traction force intensity, denoted as j.

Twenty-First Embodiment

The present embodiment differs from the twentieth embodiment in that the cell constraining device 40 in the present embodiment is made of a silicon membrane.

Specifically, referring to FIG. 9, a-c, in FIG. 9, a shows a real image of the cellular traction force detection device using a silicon membrane as the cell constraining device. In FIG. 9, (a) shows the silicon membrane, after being perforated with a laser, is adhered to the base, with each hole housing a micro-/nano-pillar. The silicon membrane restricts the morphology and migration of cells while controlling contact or adhesion between cells. In FIG. 9, (b) shows a fluorescence microscopy image of the cellular traction force detection device under light reflection, employing a silicon membrane as the cell constraint mechanism, and in FIG. 9, (c) is an enlargement image of (b) in FIG. 9. In some embodiments, each hole in the silicon membrane may be sized to fit a single cell, facilitating individual cell attachment and thereby limiting cell contact, cell morphology, and migration range.

Twenty-Second Embodiment

The present embodiment specifically provides a method for identifying cells using the cellular traction force information obtained by the system described in the twentieth embodiment.

Referring to FIG. 10, a-g, in FIG. 10, (a) is a fluorescence imaging diagram of a mixed system of healthy cells and lung non-small cell carcinoma cells; in FIG. 10, (b) shows a light reflection signal distribution image obtained by the information acquisition unit from the cellular traction force detection device; in FIG. 10, (c) is a visualization of mechanical force magnitude and distribution; in FIG. 10, (d) is an enlargement image of the representative single-cell cellular force distribution of healthy cells and lung non-small cell carcinoma cells in FIG. 10, (c); FIG. 10, (e) compares the cellular morphology of healthy cells and lung non-small cell carcinoma cells; FIG. 10, (f) compares the reflection signal intensities (reflecting cellular force) of healthy cells, lung non-small cell carcinoma cells, and mixtures of these two cell types in various proportions; and FIG. 10, (g) shows a cluster analysis diagram based on the structured cell information processed from FIG. 10, (c).

Specifically, the present embodiment uses healthy cells (Normal) and lung non-small cell carcinoma cell lines (Cancer) as detection targets, including pre-staining the cell membranes of healthy and cancer cells with two different fluorescent dyes (Dil&DIO) and then adding them in certain proportions to the same cellular traction force detection device (or to different independent cellular traction force detection devices in some embodiments).

Furthermore, the information acquisition unit (in the present embodiment, a microscope) captured images of the light reflection signals from the cellular traction force detection device (as shown in FIG. 10, b), where the high-resolution force field distribution within the two types of cells is directly rendered by the information acquisition unit into readable light intensity attenuation signals (reflecting cellular force strength) and displayed in the image (as shown in FIG. 10,c). Based on the different light attenuation levels of the two cell types shown in FIG. 10,c, a direct visual distinction between the two cell types can be found through naked eye observation (qualitative analysis).

Furthermore, the light reflection signals in FIG. 10, c are further processed by a light signal analysis device.

Specifically, the present embodiment uses ImageJ and Python analysis software (other image/data analysis software can also be used in other embodiments) to collect information from the cellular force field image in FIG. 10,c, including collecting information on the magnitude of cellular traction force at multiple points within each cell, thereby obtaining data on the magnitude of cellular traction force at multiple points within multiple cells; preprocessing the obtained cellular traction force magnitude information to form structured cell information; and analyzing the structured cell information to obtain comparative results of healthy cells and lung non-small cell carcinoma cells in terms of cellular morphology (as shown in FIG. 10,e).

The structured cell information includes the number of cells, the number of cellular features, and the information for each cell feature (e.g., in the present embodiment, cell adhesion area and cell roundness). At this stage, the structured cell information can be viewed as a two-dimensional feature matrix (Feature Matrix), where N is the number of cells, and P is the number of cellular features, here P=2, meaning the cellular features are: the magnitude of cellular traction force and the distribution of cellular traction force within the cell.

Furthermore, using the above-mentioned structured cell information as input data, a supervised image/data processing device's learning unit (comparing the different cell membrane dyes (Dil&DIO) used for pre-staining the two cell lines) learns to establish a cellular feature model, and then the model with a large amount of structured cell information from numerous cells is trained, resulting in the cluster analysis diagram as shown in FIG. 10,g. The obtained cellular feature model is applied to the classification and identification of cells of unknown type or status. It can be seen that based on the structured cellular feature data (the magnitude of cellular traction force, the distribution of cellular traction force within the cell) as the input data, the image/data processing device's identification unit can cluster and differentiate normal healthy cells and cancer cells, thereby achieving identification of cell of unknown types.

FIG. 10, e shows no statistically significant difference in the morphology between different cells (including cell adhesion area and cell roundness); FIG. 10,f demonstrates significant differences in the reflection signal intensity (reflecting cellular force) between normal and tumor cells, and a certain linear relationship between reflection signal intensity and mixing ratio when normal cells and tumor cells are mixed together in certain proportions. It is thus evident that, compared to other features of cells (e.g., cell adhesion area, cell roundness, and other morphological information in FIG. 10,e), the mechanical characteristics of cells measured by the cellular traction force detection device proposed in the present application allow for a more intuitive and accurate identification of cell status and type (quantitative and qualitative analysis).

Furthermore, the data from FIG.s 10,d and f show that tumor cells exhibit higher cellular traction force and more uneven distribution than normal cells. It is clear that once the cellular traction force is visualized in an image format, the force field characteristics of different cells can be distinctly observed with the naked eye; and further, through image analysis software, structural processing of the force field magnitude at various points within different cells allows for comprehensive analysis to obtain the cellular morphology information in FIG. 10,e, the reflection signal intensity (reflecting cellular force) in FIG. 10,f, and the cluster analysis diagram in FIG. 10,g. The present application, through comprehensive analysis of structured information on the force field of various points within cells, can cluster and quantitatively analyze different cells (e.g., healthy cells and non-small cell lung cancer cells in the present embodiment), thereby achieving precise identification of cell types.

In summary, the cell identification system including the cellular traction force detection device described in the present embodiment not only allows for direct visual differentiation for qualitative analysis but also enables more intuitive and accurate identification of cell status and type based on the measured cellular mechanical characteristics (quantitative and qualitative analysis), confirming that using the cell force field as a biomarker can better differentiate cell types.

Twenty-Third Embodiment

The present embodiment specifically provides a cellular identification system, as described in the twentieth embodiment, being applied to monitor cell vitality by using the cellular traction force information obtained.

Referring to FIG. 11, a-c, in FIG. 11, (a) is a schematic operational procedure diagram for the cell vitality detection method; FIG. 11,b compares cell vitality determined by the MTT assay and cellular traction force obtained by the cellular identification system of the present embodiment in A549 cells after treatment with various doses of 5-FU for 24 hours; FIG. 11, c compares cell vitality determined by the MTT assay and cellular traction force obtained by the cellular identification system of the present embodiment in A549 cells treated with various doses of 5-FU for different time.

Specifically, in the present embodiment, non-small cell lung cancer A549 cells are cultured on multiple cellular traction force detection devices, and then are treated with various doses of cell proliferation inhibitor 5-fluorouracil (5-FU). Subsequently, the cellular traction force at different time are monitored by using the cellular identification system described in the twentieth embodiment. Cell proliferation and cytotoxicity are monitored using the CCK-8 kit, with cell vitality measured by the MTT assay serving as a control group, yielding the data for FIG.s 11,b and c.

As shown in FIG.s 11,b and c, both cell vitality measured by the traditional MTT assay and cell vitality reflected by cellular traction force show a dose-dependent decrease, indicating a positive correlation between cellular traction force and cell vitality.

Moreover, as shown in FIG. 11,b, compared to the control group DMSO, cellular vitality decreases more significantly with different doses of 5FU treatment for 24 hours as reflected by cellular traction force, providing a more intuitive assessment of cell vitality. As depicted in FIG. 11,c, after 12 hours of treatment with 5FU, the changes in cell vitality measured by the MTT assay are not noticeable; however, a decrease in cellular traction force can be observed at earlier time before a reduction in metabolic activity is detected by the MTT assay, specifically a significant decrease at 6 hours with a 0.5 $\mu$M treatment dose and at 3 hours with a 1 $\mu$M treatment dose, thereby allowing for a more sensitive characterization of decreased cell vitality.

In summary, the present embodiment demonstrates that direct measurement of cellular traction force using the cellular identification system, including the cellular traction force detection device of the present embodiment, is a highly sensitive and effective method for assessing cellular response to drug treatment.

Twenty-fourth Embodiment

The present embodiment provides a cellular identification system, as described in the twentieth embodiment, being applied to detect cell states based on the cellular traction force information obtained.

Referring to FIG.s 12, a-f, where FIG. 12, a is a process diagram of the cell state detection method; FIG. 12,b shows the fluorescence microscopy image of M0 macrophages distinct into M1 state; FIG. 12,c shows the fluorescence microscopy image of M0 macrophages distinct into M2 state; FIG. 12,d compares the cell adhesion area of M0 macrophages, M1 state, and M2 state; FIG. 12,e compares the cell roundness of M0 macrophages, M1 state, and M2 state; and FIG. 12,f compares the cellular traction force of M0 macrophages, M1 state, and M2 state.

Specifically, the present embodiment uses macrophages as detection subjects, places these cells on micro-/nano-pillars of different independent cellular traction force detection devices and uses lipopolysaccharides (LPS) and interleukin-4 (IL4) to guide macrophages from M0 to differentiate into M1 and M2 states, with M0 state serving as the control group. After cell differentiation, FIG.s 12,b and c are captured by the information acquisition unit (microscope used in the present embodiment), and further image processing and data analysis are performed using image/data processing devices (ImageJ and Python), transforming them into structured information and analyzing to produce the data for FIG.s 12,d-f. Data from FIG.s 12,a to f indicate that there are significant differences between M0 macrophages and their distinct states of M1 and M2, both from direct observation (FIG.s 12,b and c) and structured data quantification (FIG.s 12,d-f).

Twenty-fifth Embodiment

The present embodiment specifically provides a cellular identification system, as described in the twentieth embodiment, to obtain cellular traction force information and to analyze and detect cell states based on this information.

Specifically, the present embodiment offers methods for combining multicellular aggregates on the cellular traction force detection device in various ways, with two specific methods provided:

The first method involves setting a culture medium on the micropillars of the cellular traction force detection device and transplanting cells into the culture medium on the micropillars to cultivate multicellular aggregates. In some implementations, this method allows for real-time monitoring of cell culture processes under visual representation of cellular traction force information, thereby being applicable for studying the effects of chemical, biological, and physical external stimuli such as culture media and drugs on cell growth.

The second method involves directly attaching pre-cultivated multicellular aggregates to the micropillars of the cellular traction force detection device for detection.

More specifically, the present embodiment provides a method for culturing tumor cell aggregates on the cellular traction force detection device for drug sensitivity testing, including the following steps:

S1, Generation of tumor cell aggregates: FN 50 $\mu$g/mL is coated on the top of the micropillars of the cellular traction force detection device and sterilized with UV light for 30 minutes. Breast cancer cells MCF-7 (approximately $1\times10^5$ to $9\times10^5$ cells) are seeded on the surface of the micropillars of the cellular traction force detection device (number of micropillars not limited) and then cultured in 3dGRO™ Spheroid Medium (S3077) for more than three days to guide the formation of tumor cell aggregates.

S2, Applying the generated tumor cell aggregates to 5-Fu drug sensitivity experiments: 200 $\mu$M of 5-Fu is added to the tumor cell aggregates and cultured for one day. In the experiment, cellular traction force detection devices with tumor cell aggregates (along with the culture medium) before and after the addition of 5-Fu are monitored using the information acquisition unit (CCD sensor) within the cellular identification device to capture light reflection signals. The cell force distribution images are obtained using optical image analysis software (Image J), as shown in FIG.s 13,a and b.

Refer to FIG.s 13,a-b, wherein FIG. 13,a shows characterization images of tumor cell aggregates with/without the effect of 5-Fu in the first form, and FIG. 13,b for the second form. From left to right, these images include mixed images of cell membrane fluorescence and reflection signals 1, light reflection signals 2, nuclei 3, cell membranes 4, and cell force visualization images processed by optical image analysis software (Image J) 5. Due to cellular heterogeneity, tumor cells differ, and aggregates form in various shapes, leading to different attachment forms of cell aggregates. The present embodiment selects two representative forms for cell morphology detection: the first form represents larger cells adhered together, and the second form represents a cluster of smaller cells adhered together.

FIG.s 13,a-b demonstrate that after treating cells with anti-tumor drugs to reduce their vitality, reflection signals significantly weaken; changes in cellular traction force before and after 5-Fu treatment indicate distinct drug sensitivities between the two forms of tumor cell aggregates. Thus, the cellular traction force detection device of the present application can measure the cellular traction force of multicellular aggregates (such as tumor aggregates), monitor the vitality state of cell aggregates, and distinguish different cell morphologies.

In the context of the present application, multicellular aggregates refer to cell groups formed when cells, the basic structural and functional units of organisms, reproduce or differentiate to join together, including tumor aggregates obtained from in vitro or in vivo cultures.

It should be noted that, although the embodiments above have been described, they do not limit the scope of the patent protection of the present application. Therefore, changes and modifications made to the embodiments described herein, or equivalent structural or procedural transformations made based on the innovative concepts of the present application and specification and drawings thereof, directly or indirectly applied in other related technical fields, are included within the scope of the present application.

**Claims**

1.  A cell identification method, **characterized by** comprising:

    acquiring cell information, wherein the cell information comprises cellular traction force information at a point within a cell obtained via a cellular mechanical sensor, and the cellular traction force information comprises a magnitude of the cellular traction force at the point;
    preprocessing the acquired cell information to generate structured cell information, wherein the structured cell information comprises the number of cells, the number of cell features, and feature information for each cell feature;
    inputting the structured cell information into a machine learning model established through supervised, unsupervised, or semi-supervised learning; and
    applying the machine learning model to classify or cluster cells of unknown types or states.

2.  The method according to claim 1, wherein the cellular traction force information further comprises a direction of the cellular traction force at the point.

3.  The method according to claim 1, wherein the cellular traction force information further comprises changes in the magnitude or direction of the cellular traction force at the point over a time interval.

4.  The method according to claim 1, wherein the cell information further comprises cell morphology information.

5.  The method according to claim 1, wherein the cell information is obtained by performing cell confining operations on the cell.

6.  A cell identification device, **characterized by** comprising:

    an information acquisition unit configured to acquire cell information, wherein the cell information comprises cellular traction force information at a point within a cell obtained via a cellular mechanical sensor, and the cellular traction force information comprises a magnitude of the cellular traction force at the point;
    a preprocessing unit configured to preprocess the cell information to generate structured cell information, wherein the structured cell information comprises the number of cells, the number of cell features, and feature information for each cell feature;
    a learning unit configured to use the structured cell information as input data for establishing a cell feature model via supervised, unsupervised, or semi-supervised learning; and
    an identification unit configured to apply the cell feature model to classify or cluster cells of unknown types or states.

7. The cell identification device according to claim 6, wherein the cellular traction force information further comprises a direction of the cellular traction force at the point.

8. The cell identification device according to claim 6, wherein the cellular traction force information further comprises changes in the magnitude and/or direction of the cellular traction force at the point over a time interval.

9. The cell identification device according to claim 6, wherein the cell information further comprises cell morphology information.

10. The cell identification device according to claim 6, wherein the cell information is obtained by performing cell confining operations on the cell.

11. A cell identification system, **characterized by** comprising the cellular mechanical sensor and the cell identification device as described in any one of claims 6 to 10.

12. The system according to claim 11, further comprising a cell morphology information acquisition device configured to acquire cell morphology information.

13. The system according to claim 12, wherein the cell morphology information acquisition device comprises a microscope camera or a video microscope.

14. The system according to claim 11, further comprising a cell confinement device configured to perform cell confining operations.

15. A method for detecting cell states, **characterized by** comprising:

acquiring cellular traction force information by the cell identification method as described in any one of claims 1 to 5, or by a cell identification device as described in any one of claims 6 to 10, or by a cell identification system as described in any one of claims 11 to 14;
analyzing and determining cell states based on the cellular traction force information;
wherein the cell states comprise cell adhesion, cell vitality, cell differentiation/activation, cell proliferation, and/or cell migration.

FIG. 1

FIG. 2

FIG. 3

FIG. 4

CELL IDENTIFICATION SYSTEM

CELLULAR MECHANICAL SENSOR (CELLULAR
TRACTION FORCE DETECTION DEVICE) —— 10

CELL IDENTIFICATION DEVICE —— 20

INFORMATION ACQUISITION UNIT —— 1

PREPROCESSING UNIT —— 2

LEARNING UNIT —— 3

IDENTIFICATION UNIT —— 4

CELL MORPHOLOGY INFORMATION
ACQUISITION DEVICE —— 30

CELL CONFINEMENT DEVICE —— 40

FIG. 5

light-reflective layer 1031

micropillar 103

base 101

FIG. 6

light-reflective layer 1031

micropillar 103

cellular traction force detection device 10

base 101

Spectroscope 104

light signal generation device 102

light signal detection device 1

preprocessing unit 2

learning unit 3

identification unit 4

image/data processing device 201

cell identification device 20

FIG. 7

Pillar Deformation, Subject to Cellular Mechanical Force

a) Reflective Layer (metal) Cell

Illumination Reflection

b)

LIGHT SIGNAL GENERATION DEVICE

INFORMATION ACQUISITION UNIT

IMAGE/DATA PROCESSING DEVICE

c)

Bright field reflection (with cell): I

FFT generated reflection (no cell): $I_0$

$I_0$ subtract with I

Applied with force index: j

FIG. 8

a)

Microwell

b)

A549 cell membrane (DiI)
Light Reflection

c)

FIG. 9

a)

Lung cancer cell
Normal cell

b)

c)

0 .      30    Mechanical Force (nN)
(Traction force)

d)

Normal

Cancer

e)

ns

ns

Cell area (μm²)

Cell circularity

Normal   Cancer

Normal   Cancer

f)

Reflective Intensity

16000
14000
12000
10000
8000

Normal Cancer Mix1 Mix2

Mix1: 90% Normal + 10% Cancer
Mix2: 80% Normal + 20% Cancer

g)

○ Cancer   ● Normal

PC2

PC1

FIG. 10

FIG. 11

FIG. 12

FIG. 13

## INTERNATIONAL SEARCH REPORT

International application No.

**PCT/CN2022/121340**

### A. CLASSIFICATION OF SUBJECT MATTER

G06V 20/69(2022.01)i

According to International Patent Classification (IPC) or to both national classification and IPC

### B. FIELDS SEARCHED

Minimum documentation searched (classification system followed by classification symbols)

G06V, G06K

Documentation searched other than minimum documentation to the extent that such documents are included in the fields searched

Electronic data base consulted during the international search (name of data base and, where practicable, search terms used)

CNTXT, ENTXT, ENTXTC, DWPI, CNKI, CNABS: 细胞, 传感器, 牵引力, 预处理, 模型, 机器学习, 深度学习, 特征, 聚类, 分类, 结构, 监督, cell, sensor, traction, pretreatment, model, machine learning, deep learning, character, clustering, class+, structure, supervise

### C. DOCUMENTS CONSIDERED TO BE RELEVANT

| Category* | Citation of document, with indication, where appropriate, of the relevant passages | Relevant to claim No. |
|---|---|---|
| Y | CN 110647874 A (BEIJING XIAOYING TECHNOLOGY CO., LTD.) 03 January 2020 (2020-01-03)<br>description, paragraphs 2-44 | 1-15 |
| Y | US 2019317050 A1 (UNIV HUNAN AGRICULTURAL) 17 October 2019 (2019-10-17)<br>description, paragraphs 4 and 21-32 | 1-15 |
| Y | CN 111666895 A (SHANGHAI TONGJI HOSPITAL) 15 September 2020 (2020-09-15)<br>description, paragraphs 2-368 | 1-15 |
| Y | CN 104359876 A (XIAMEN UNIVERSITY) 18 February 2015 (2015-02-18)<br>description, paragraphs 3-47 | 1-15 |

☐ Further documents are listed in the continuation of Box C.  ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **04 November 2022** | **03 January 2023** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.

**PCT/CN2022/121340**

| Patent document cited in search report | | | Publication date (day/month/year) | Patent family member(s) | | | Publication date (day/month/year) |
|---|---|---|---|---|---|---|---|
| CN | 110647874 | A | 03 January 2020 | CN | 110647874 | B | 28 August 2020 |
| | | | | WO | 2021104410 | A1 | 03 June 2021 |
| US | 2019317050 | A1 | 17 October 2019 | JP | 2019530442 | A | 24 October 2019 |
| | | | | EP | 3505925 | A1 | 03 July 2019 |
| | | | | WO | 2018041060 | A1 | 08 March 2018 |
| | | | | CN | 106404915 | A | 15 February 2017 |
| | | | | AU | 2017319889 | A1 | 18 April 2019 |
| | | | | US | 11029285 | B2 | 08 June 2021 |
| | | | | EP | 3505925 | A4 | 08 April 2020 |
| | | | | AU | 2017319889 | B2 | 06 January 2022 |
| | | | | JP | 6851651 | B2 | 31 March 2021 |
| | | | | CN | 106404915 | B | 19 February 2019 |
| CN | 111666895 | A | 15 September 2020 | None | | | |
| CN | 104359876 | A | 18 February 2015 | CN | 104359876 | B | 10 July 2018 |

Form PCT/ISA/210 (patent family annex) (January 2015)